(19) Europäisches Patentamt
European Patent Office
Office européen des brevets

(11) Veröffentlichungsnummer: **0 046 863**
**B1**

# (12) EUROPÄISCHE PATENTSCHRIFT

(45) Veröffentlichungstag der Patentschrift: **29.06.83**

(51) Int. Cl.³: **C 07 C 99/00,** C 07 C 101/80

(21) Anmeldenummer: **81105564.9**

(22) Anmeldetag: **15.07.81**

**(54) Verfahren zur Herstellung von 1-Aminoanthrachinon-2-carbonsäure.**

(30) Priorität: **29.08.80 DE 3032547**

(43) Veröffentlichungstag der Anmeldung:
**10.03.82 Patentblatt 82/10**

(45) Bekanntmachung des Hinweises auf die Patenterteilung:
**29.06.83 Patentblatt 83/26**

(84) Benannte Vertragsstaaten:
**BE CH DE FR GB LI**

(56) Entgegenhaltungen:
**DE-A-2 228 660**
**DE-A-2 531 259**

**HOUBEN-WEYL «Methoden der organischen Chemie», 4. Auflage, Band VII/3c, 1979, GEORG THIEME VERLAG, Stuttgart, Seiten 158 bis 161**

(73) Patentinhaber: **BASF Aktiengesellschaft, Carl-Bosch-Strasse 38, D-6700 Ludwigshafen (DE)**

(72) Erfinder: **Kohler, Rolf-Dieter, Dr., Amselweg 3, D-6803 Edingen (DE)**
Erfinder: **Hagen, Helmut, Dr., Max-Slevogt-Strasse 17E, D-6710 Frankenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Verfahren zur Herstellung von 1-Aminoanthrachinon-2-carbonsäure

Die Erfindung betrifft ein Verfahren zur Herstellung von 1-Aminoanthrachinon-2-carbonsäure durch Umsetzung von 1-Nitroanthrachinon-2-carbonsäure mit Formaldehyd in Gegenwart alkalischer Verbindungen.

Es ist aus Houben-Weyl, Methoden der Organischen Chemie, Band 7/3c, Seiten 158 bis 161 bekannt, dass man 1-Nitroanthrachinon durch Reduktion, z.B. durch Dinatriumsulfid und Natronlauge; Zinn-II-chlorid in verdünnter Natronlauge; Glucose und Natronlauge; Hydrazin udn Natronlauge; mit Ammoniak unter Druck; in 1-Aminoanthrachinon verwandeln kann. Es wird auf die Empfindlichkeit zahlreicher Nitroverbindungen und die besondere Selektivität jedes Reduktionsmittels und damit auf die Gefahr der Bildung von Nebenprodukten hingewiesen. Obwohl die Ausbeute und Reinheit der nach dieser Methode gewonnenen 1-Aminoanthrachinon-2-carbonsäure für die weiteren Synthesestufen befriedigt, ist der Einsatz grosser Mengen an überschüssigem Ammoniak und der dafür notwendigen Druckgefässe sehr aufwendig. Alle diese Verfahren sind im Hinblick auf einfachen und wirtschaftlichen Betrieb, hohe Ausbeute und Reinheit des Endstoffs unbefriedigend.

Es wurde nun gefunden, dass man 1-Aminoanthrachinon-2-carbonsäure durch Reduktion von 1-Nitroanthrachinonen vorteilhaft erhält, wenn man 1-Nitroanthrachinon-2-carbonsäure mit Formaldehyd in Gegenwart von alkalischen Verbindungen umsetzt.

Die Umsetzung kann für den Fall der Verwendung von Natronlauge durch die folgenden Formeln wiedergegeben werden:

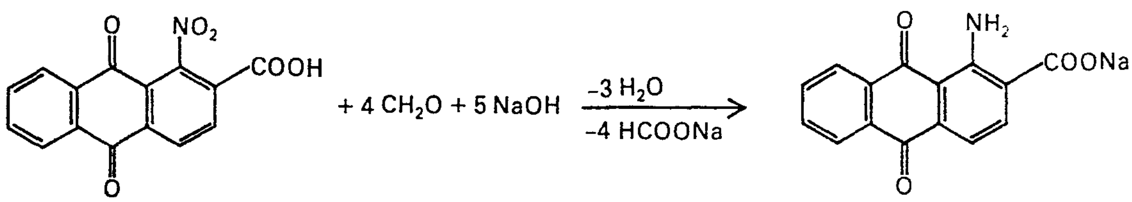


Im Hinblick auf den Stand der Technik liefert das Verfahren nach der Erfindung auf einfacherem und wirtschaftlicherem Wege 1-Aminoanthrachinon in besserer Ausbeute und Reinheit. Da die erfindungsgemässen Ausgangsstoffe in der Regel drucklos umgesetzt werden, werden alle Nachteile einer Umsetzung unter Druck vermieden. Alle diese vorteilhaften Ergebnisse sind im Hinblick auf die bekannten Verfahren überraschend. Es konnte nicht erwartet werden, dass Formaldehyd in stark alkalischer Lösung ein wirksames Reduktionsmittel darstellt, da man annehmen musste, dass unter den angewandten Reaktionsbedingungen Formaldehyd bevorzugt nach der Cannizzaro-Reaktion zu Methanol und Natriumformiat reagiert.

1-Nitroanthrachinon-2-carbonsäure kann als freie Säure oder als Salz, vorzugsweise Alkalisalz wie das Natriumsalz, umgesetzt werden. Formaldehyd kann gasförmig oder in der Regel jedoch in Form seiner wässrigen Lösung verwendet werden. Man kann auch Stoffe, die unter den Reaktionsbedingungen Formaldehyd abgeben, verwenden, z.B. Paraformaldehyd, Trioxan, Polyoxymethylene wie Tetraoxymethylen, Hexamethylentetramin. Die wässrigen Formaldehydlösungen enthalten zweckmässig 30 bis 55 Gewichtsprozent, vorzugsweise 35 bis 45 Gewichtsprozent Formaldehyd (berechnet 100 Prozent). Formaldehyd kann mit 1-Nitroanthrachinon-2-carbonsäure in stöchiometrischer Menge oder im Überschuss, zweckmässig in einer Menge von 1 bis 20, insbesondere 2 bis 12 Mol Formaldehyd, bezogen auf 1 Mol 1-Nitroanthrachinon-2-carbonsäure, umgesetzt werden.

Die Umsetzung wird in Gegenwart einer alkalischen Verbindung, vorteilhaft in einer Menge von 5 bis 20, vorzugsweise von 10 bis 15 Äquivalenten alkalischer Verbindung, bezogen auf ein Mol 1-Nitroanthrachinon-2-carbonsäure, durchgeführt. Bevorzugte alkalische Verbindungen sind Natrium- und Kaliumverbindungen, insbesondere Hydroxide, Carbonate sowie entsprechende Gemische. Es kommen z.B. als basische Verbindungen in Frage: Kaliumhydroxid, Natriumhydroxid, Kaliumcarbonat, Natriumcarbonat.

Die Umsetzung des Ausgangsgemischs wird zweckmässig bei einer Temperatur von 40 bis 120°C, vorzugsweise 60 bis 100°C, unter Überdruck oder Unterdruck oder bevorzugt drucklos, kontinuierlich oder diskontinuierlich durchgeführt. Das Lösungsmittel ist vorteilhaft Wasser und wird zweckmässig in einer Menge von 50 bis 1000, vorzugsweise von 80 bis 500 Gewichtsprozent, bezogen auf 1-Nitroanthrachinon-2-carbonsäure, verwendet.

Die Reaktion kann wie folgt durchgeführt werden: Das Gemisch von 1-Nitroanthrachinon-2-carbonsäure und wässriger Formaldehydlösung, wird zusammen mit der alkalischen Verbindung während 0,5 bis 4 Stunden bei der Umsetzungstemperatur gehalten. Nun wird das Gemisch mit Säure, bevorzugt Schwefelsäure, Salzsäure, Ameisensäure, Essigsäure neutralisiert. Der Endstoff wird dann in üblicher Weise aus dem Gemisch, z.B. durch Filtration, isoliert.

Die nach dem Verfahren der Erfindung hergestellte 1-Aminoanthrachinon-2-carbonsäure ist ein wertvoller Ausgangsstoff für die Herstellung von Farbstoffen und Schädlingsbekämpfungsmitteln. Bezüglich der Verwendung wird auf Ullmanns Encyklopädie der technischen Chemie (7. Auflage), Band 3, Seiten 676 bis 681, verwiesen.

Die in dem folgenden Beispiel aufgeführten Teile bedeuten Gewichtsteile.

Beispiel

Eine 60°C warme Mischung aus 320 Teilen Natriumsalz der 1-Nitroanthrachinon-2-carbonsäure und 300 Teilen Formaldehyd (40-gewichtsprozentige, wässrige Lösung) in 2900 Teilen Wasser gibt man innerhalb von 30 Minuten zu 2500 Teilen einer auf 98°C erwärmten 5-n-Natronlauge. Die Mischung wird nach zweistündigem Rühren bei 98°C auf 40°C gekühlt, mit 1000 Teilen 30-gewichtsprozentiger Schwefelsäure neutralisiert und weitere 2 Stunden bei 98°C gerührt. Anschliessend wird der Niederschlag heiss filtriert, mit heissem Wasser gewaschen und bei 80°C im Vakuum getrocknet. Man erhält 256 Teile 1-Aminoanthrachinon-2-carbonsäure, was einer Ausbeute von 95,8% der Theorie entspricht. Der Reinheitsgehalt liegt bei 95,1 Prozent.

**Patentanspruch**

Verfahren zur Herstellung von 1-Aminoanthrachinon-2-carbonsäure durch Reduktion von 1-Nitroanthrachinonen, dadurch gekennzeichnet, dass man 1-Nitroanthrachinon-2-carbonsäure mit Formaldehyd in Gegenwart von alkalischen Verbindungen umsetzt.

**Claim**

A process for the preparation of 1-aminoanthraquinone-2-carboxylic acid by reducing 1-nitroanthraquinones, wherein 1-nitroanthraquinone-2-carboxylic acid is reacted with formaldehyde in the presence of an alkaline compound.

**Revendication**

Procédé pour la préparation d'acide 1-amino-anthraquinone-2-carboxylique par réduction de 1-nitro-anthraquinones, caractérisé en ce qu'on fait réagir l'acide 1-nitro-anthraquinone-2-carboxylique avec le formaldéhyde en présence de composés alcalins.